# EUROPEAN PATENT APPLICATION

(11) **EP 1 655 002 A2**
(43) Date of publication of application: **10.05.2006**
(21) Application number: 06002587.1
(22) Date of filing: 12.01.1999
(51) Int. Cl.: A61F 2/06

(54) **Transmyocardial coronary artery bypass and revascularization**

(30) Priority: 30.01.1998 US 16485
(62) Divisional of application: 99902171.0
(71) Applicant: WILK PATENT DEVELOPMENT CORPORATION, New York, NY 10003 (US)
(72) Inventor: Wilk, Peter J., New York, NY 10023 (US)
(74) Representative: Grünecker, Kinkeldey, Stockmair & Schwanhäusser Anwaltssozietät

(57) **Abstract**

The present invention relates to a stent having a collapsed configuration and an expanded configuration, said expanded configuration having an arcuate form to provide a curved flow path for blood upon implantation of said stent into a myocardium of a patient.

## Description

### Background of the Invention

This invention relates to a method for effectuating a coronary artery bypass. Coronary arteries frequently become clogged with plaque which at the very least impairs the efficiency of the heart's pumping action and can lead to heart attack. The conventional treatment for a clogged coronary artery is a coronary by-pass operation wherein one or more venous segments are inserted between the aorta and the coronary artery. The inserted venous segments or transplants by-pass the clogged portion of the coronary artery and thus provide for a free or unobstructed flow of blood to the heart.

Such conventional coronary artery by-pass surgery is expensive, time-consuming, and traumatic to the patient. Hospital stay subsequent to surgery and convalescence are prolonged. Other more advanced therapies have been proposed which are minimally invasive and possibly more effective in the long run. U.S. Patents Nos. 5,287,861 and 5,409,019 to Wilk, for example, disclose a coronary-bypass method using a collapsible stent made of a biocompatible material and having an inherent spring bias tending to form the stent into a substantially tubular opened configuration. The stent is disposed in a wall of a patient's heart so that the stent extends between the left ventricle of the heart and a coronary artery. Upon placement, the stent opens and allows blood to flow from the left ventricle into the coronary artery during diastole and is closed by heart contraction during systole.

U.S. Patent No. 5,429,144 to Wilk discloses the disposition of a stent in the myocardium so that the stent extends only in the myocardium. The stent may extend only partially through the myocardium, from the left ventricle of the heart or from a coronary artery, upstream of a vascular obstruction. Alternatively, the stent may extend completely through the myocardium to establish a blood flow path from the left ventricle to a coronary artery, downstream of a vascular obstruction.

Where stents are used in the Wilk cardiac revascularization techniques to guide blood from the left ventricle, the stents are generally designed to lock upon opening from collapsed insertion configurations. Such stents enable the infusion of blood into the myocardium during systole. The stents may be provided with one-way vales to prevent a backflow of blood during diastole.

Another therapeutic method for revitalizing an ailing heart is transmyocardial revascularization. In this technique, holes or formed in the myocardium, generally-during open heart surgery. The holes traverse the myocardium from the ventricles through the epicardium. After completion of this procedure, the heart muscle functions during systole to close the outer ends of the holes, with blood being forced under high pressure into the inner ends of the holes from the left ventricle.

### Objects of the Invention

An object of the present invention is to provide a new method for performing a coronary artery by-pass operation.

Another object of the present invention is to provide such a method which is less invasive and less traumatic to the patient than conventional by-pass surgery.

An additional object of the present invention is to provide such a method which is less expensive than conventional by-pass surgery.

A more particular object of the present invention is to provide such a method which requires no incision through the chest wall.

Yet another object of the present invention is to provide a catheter assembly for use in performing the method of the invention.

These and other objects of the present invention will be apparent from the drawings and detailed descriptions herein.

### Summary of the Invention

The present invention provides methodology and related medical devices for effectively bypassing a blocked or partially blocked coronary artery and providing oxygenated blood to the myocardium. In accordance with the present invention, a coronary artery bypass method utilizes a shunt member. An upstream end portion of the shunt member is disposed in the myocardium of a patient's heart so that the upstream end portion communicates with the left ventricle of the patient's heart. An opposite or downstream end portion of the shunt member is placed in communication with a coronary artery of the patient downstream of a blockage in the coronary artery and so that an intermediate or middle portion of the shunt member is disposed in an intrapericardial space of the patient, outside of the myocardium and outside of the coronary artery.

The downstream end portion of the shunt is inserted into the coronary artery or, alternatively, attached to a generally anterior wall of the coronary artery.

Where the downstream end portion of the shunt is attached to the anterior wall of the coronary artery, the method further comprises forming an aperture in the anterior wall of the coronary artery after attaching of the downstream end portion of the shunt member to the anterior wall, thereby opening communication between the shunt member and the coronary artery. The shunt member is preferably delivered intravascularly into the left ventricle of the patient's heart. The downstream end portion of the shunt member is then passed completely through the myocardium and the intrapericardial space to the anterior wall of the coronary artery. The aperture in the coronary artery is formed by inserting a free end portion of an incising instrument intravascularly and through the shunt member after disposition of the upstream end portion of the shunt member in the myocardium and after attaching of the downstream end portion of the shunt member to the coronary artery. The incising instrument is operated, after inserting thereof, to perforate the anterior wall of the coronary artery.

The incising instrument may be a laser instrument including an optical fiber. The incising instrument is operated in part by transmitting monochromatic or laser radiation through the optical fiber to the anterior wall of the coronary artery.

The method utilizing the shunt member further comprises forming a passageway through the myocardium prior to the disposing of the upstream end portion of the shunt member in the myocardium. The passageway is formed by inserting a surgical instrument intravascularly into the left ventricle of the patient and operating the instrument from outside the patient to bore or tunnel through the myocardium. The upstream end portion of the shunt member is disposed in the passageway and subsequently the downstream end portion of the shunt member is placed in communication with the coronary artery of the patient is performed subsequently to the disposing of the shunt member in the passageway.

The shunt member may be deployed in a pericardioscopic operation wherein pericardioscopic surgical instruments are operated from outside the patient to manipulate the downstream end portion of the shunt member and to place the downstream end portion of the shunt member into communication with the coronary artery of the patient after passing of the downstream end portion of the shunt member through the passageway in the myocardium.
Where the downstream end portion of the shunt member is inserted into the coronary artery, the sequence of operations is similar to the case where the shunt member is attached to the anterior wall of the coronary artery. The shunt member is delivered intravascularly into the left ventricle of the patient's heart and subsequently the downstream end portion of the shunt member is passed through the myocardium; the downstream end portion of the shunt member is then inserted into the coronary artery. In this case, as well, the shunt member may be deployed in a pericardioscopic operation wherein pericardioscopic surgical instruments are operated from outside the patient to place the downstream end portion of the shunt member in communication with the coronary artery.

Generally, in the above-described procedure, the downstream end portion of the shunt member communicates with the coronary artery downstream of a blockage. During systole, blood travels from the patient's left ventricle through the shunt member to the coronary artery and then to the myocardium along natural vessels. It may be necessary, in some patients, to provide two or more shunt members, depending on the number of blockages and their locations along the coronary artery.

The shunt member comprises a generally tubular member having a length greater than a width of the myocardium. The shunt member is made of a biocompatible material such as polyethylene or GORTEXT™ and is flexible at least along the middle or intermediate portion thereof. Accordingly, the intermediate or middle portion of the shunt member may be bent into an arc to facilitating the formation of a proper junction between the downstream end portion of the shunt member and the coronary artery of the patient. The tubular shunt member may be provided with a one-way valve preventing back flow of blood from the coronary artery into the ventricle.

In a specific embodiment of the invention, the upstream end portion of the tubular shunt member is wider than the downstream end portion.

As discussed above, an upstream end portion of a generally tubular shunt member may be disposed in a myocardium of a patient's heart so that the upstream end portion communicates with a left ventricle of the patient's heart, while a downstream end portion of the shunt member is inserted into a coronary artery of the patient downstream of a blockage in the coronary artery so that the downstream end portion is disposed inside the coronary artery. In a variation of the present invention, the shunt member is deployed so as to be disposed only inside the myocardium and the coronary artery. In contrast to the above described methodology, no portion of the shunt member lies in the intrapericardial space. In this variation of the method, the shunt member is again delivered intravascularly into the left ventricle of the patient's heart, with the downstream end portion being passed through the myocardium. However, in this variation, the downstream end portion is inserted directly into the coronary artery through a posterior wall thereof in contact with the myocardium.

A method for performing a myocardial revascularization comprises, in accordance with another embodiment of the present invention, forming a passageway at least partially through a myocardium of a patient from an outer surface of the patient's heart, and performing a surgical operation at an outer end of the passageway to permanently close the passageway at the outer end. In a particular implementation of this embodiment of the invention, the passageway includes a portion extending though a posterior wall of a coronary artery and is produced by forming an aperture in an anterior wall of the coronary artery and forming the passageway in substantial alignment with the aperture. In this case, the closure of the passageway is effectuated particularly by closing the aperture in the anterior wall of the coronary artery. The closing of the aperture in the anterior wall of the coronary artery may be effectuated by one of more of several techniques, including suturing, plugging, and laser coagulation. To reinforce the closure of the artery wall, a brace may be placed over the closure. The brace may take the form of a biocompatible patch attached to the heart via suturing or laser welding.

Pursuant to another feature of a myocardial revascularization technique in accordance with the present invention, a stent is inserted into the passageway formed at least partially through he patient's myocardium. The inserting of the stent is preferably performed prior to the performing of the surgical operation to close the passageway at the outer end. The myocardial revascularization technique, including the insertion of the stent, may be performed in open heart surgery or in a pericardioscopic operation. In either case, the aperture in the anterior wall of the coronary artery and the passageway in the myocardium are formed by operating an instrument taken from the group consisting of a surgical drill and a surgical laser.

Where in a myocardial revascularization technique in accordance with the present invention, the passageway is formed to communicate at an inner end with a left ventricle of the patient, the passageway may communicate at an outer end with the coronary artery or, alternatively, may terminate in the myocardium after closure of the outer end of the passageway. In the former case, blood flows from the left ventricle through the passageway, the coronary artery and blood vessels communicating with the coronary artery. In the latter case, the myocardium is revascularized directly by the passageway, rather than indirectly through the coronary artery and its tributaries.

In a myocardial revascularization technique in accordance with the present invention, the passageway may be one of a plurality of similarly formed passageways extending from the coronary artery into the myocardium of the patient. Each passageway is produced by forming a plurality of openings in the anterior wall of the coronary artery and forming the passageways in alignment with respective ones of the openings. The passageways are effective closed from the external environment (the intrapericardial space) by closing the openings in the anterior wall of the coronary artery.

Where a myocardial passageway formed in accordance with the present invention does not extend through or into a coronary artery, the closure of the passageway is effectuated on an epicardium of the patient.

A stent for a coronary artery bypass or myocardium revascularization procedure in accordance with the present invention has a collapsed configuration and an expanded configuration. The expanded configuration may have an arcuate form, to provide a curved flow path for blood upon implantation of the stent into a myocardium of a patient. This curved flow path smoothly redirects blood flow and minimizes possible adverse effects that the impulsive force of the blood might have on the patient's coronary artery and other layers of heart tissue. The stent may have a one-way valve for preventing retrograde flow of blood.

Another stent in accordance with the present invention has a collapsed configuration and an expanded configuration and is provided with a sensor and means for transmitting signals from the sensor to a receiver external to the stent. The sensor is taken from the group consisting of a pressure sensor and a flow sensor.

A method in accordance with the present invention greatly reduces the expense of coronary surgery, as well as the trauma to the patient and the convalescence required after the by-pass operation.

### Brief Description of the Drawings

Figs. 1A-1E are schematic cross-sectional views of a human heart, showing successive steps in a shunt-utilizing transmyocardial coronary artery bypass operation in accordance with the present invention.
Fig. 2 is a schematic cross-sectional view of a human heart showing an alternative shunt to that used in the operation of Figs. 1A-1E.
Fig. 3 is a schematic partial cross-sectional view, on a larger scale, showing a modification of the coronary artery bypass produced by the operation of Figs. 1A-1E.
Fig. 4 a schematic cross-sectional view of a human heart showing a modification of the coronary artery bypass operation depicted in Figs. 1A-1E.
Fig. 5 is a schematic partial cross-sectional view, on a larger scale, showing a variation of the coronary artery bypass of Fig. 4.
Fig. 6A is a schematic partial cross-sectional view of another coronary artery bypass in accordance with the present invention, showing a shunt with a one-way valve opened during systole.
Fig. 6B is a schematic partial cross-sectional view similar to Fig. 6A, illustrating the shunt of Fig. 6A with the valve closed during diastole.
Fig. 7 is a schematic cross-sectional view of a human heart showing instrumentation used for implanting the shunt of Figs. 6A and 6B.
Fig. 8 is a schematic partial cross-sectional view of an arcuate stent with a one-way valve utilized in a further coronary artery bypass in accordance with the present invention.
Fig. 9 is a block diagram of operational components in a stent in accordance with the present invention, with feedback as to operational parameters.
Figs. 10A-10C are schematic cross-sectional views of a human heart, showing successive steps in a stent-utilizing transmyocardial coronary artery bypass operation in accordance with the present invention.
Fig. 11 is a schematic cross-sectional view similar to Fig. 10C, showing three transmyocardial stents implanted pursuant to the procedure of Figs. 10A-10C.
Fig. 12 is a schematic partial cross-sectional view of an artificial myocardial revascularization in accordance with the present invention, showing a plurality of stents extending from a coronary artery partially into the myocardium.
Fig. 13 is a schematic front elevational view of a human heart, showing an improvement in the myocardial revascularization of Fig. 12.
Figs. 14A and 14B are schematic cross-sectional views of a human heart, showing successive steps in an artificial myocardial revascularization procedure in accordance with the present invention, resulting in a plurality of stents extending from a left ventricle of the heart at least partially into the myocardium.
Fig. 15 is a schematic partial cross-sectional view of a human heart, illustrating a modification to the artificial myocardial revascularization of Fig. 14B.
Fig. 16 is a schematic partial cross-sectional view of a human heart, illustrating a heart provided in a left ventricle with implants or plugs in accordance with the present invention.

In the drawings, the same reference designations are used to designate the same objects. The word "distal" when used herein designates an instrument end which is spaced from the surgeon, radiologist or other operator. The physical relation of the instrument to the patient is not determinative.

### Description of the Preferred Embodiment

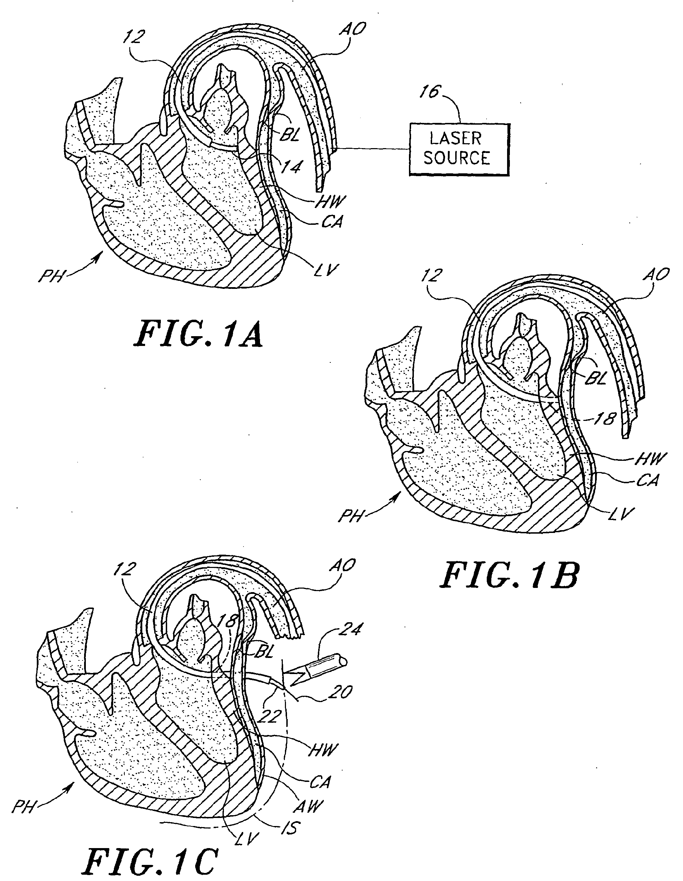

In a transmyocardial coronary artery bypass operation illustrated in Figs. 1A- 1E, a catheter 12 is inserted over a guidewire (not illustrated) through the vasculature of a patient and particularly through the aorta AO into the left ventricle LV of the patient's heart PH. Upon arrival a distal end of catheter 12 in left ventricle LV, the guidewire is withdrawn and a surgical incising instrument 14 such as a light-transmitting optical fiber is inserted through catheter 12. The catheterization procedure is monitored via conventional radiographic techniques or, alternatively, via a CAT scanner or MRI machine.

Upon ejection of a distal tip of optical fiber 14 from catheter 12 into ventricle LV, the fiber tip is placed into contact with a heart wall HW of the patient at a predetermined location downstream of an arterial blockage BL in the coronary artery CA of the patient, as illustrated in Fig. 1A. A laser source 16 is then activated to transmit monochromatic electromagnetic radiation along optical fiber 14 to heart wall HW. The distal end of fiber 14 is pushed through heart wall, with the radiation being continuously or periodically transmitted through optical fiber 14, thereby forming a transmyocardial passageway 18 in heart wall HW (Fig. 1B).

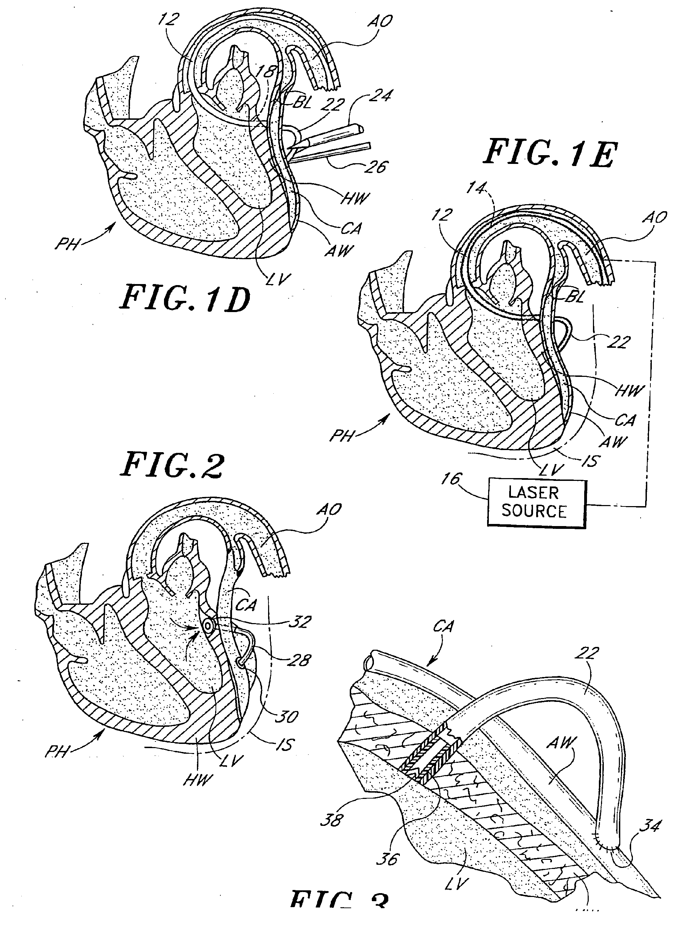

After the formation of passageway 18, optical fiber 14 is withdrawn from catheter 12 and replaced with a guidewire 20 (Fig. 1C). In addition, catheter 12 is pushed in a forward direction through passageway 18 so that a distal end portion of the catheter extends outwardly from passageway 18 into an intrapericardial space IS. A shunt 22 made of flexible biocompatible material such as polyethylene or GORTEX™ is then passed over guidewire 20 and through catheter 14. At this juncture, a forceps instrument 24 (Figs. 1C and 1D) inserted into the patient via a pericardioscopic cannula or port (not shown) or through an open incision (not shown) is used to grasp shunt 22 and direct a free end of the shunt to an anterior wall AW of coronary artery CA, as illustrated in Fig. 1D. A laser instrument 26 is then used to attach the free end of shunt 22 to the anterior wall AW of coronary artery CA. At this point in the operation, there is no avenue of communication between left ventricle LV and coronary artery CA.

After the attachment of shunt 22 to anterior wall AW of coronary artery CA, optical fiber 14 is again inserted through catheter 12 and through shunt 22 to anterior wall AW of coronary artery CA. Laser source 16 is temporarily activated to form an aperture in anterior wall AW of coronary artery CA inside shunt 22, thereby establishing a transmyocardial coronary artery bypass path from left ventricle LV into the coronary artery downstream of blockage BL. After the formation of the aperture in coronary artery CA, fiber 14 is withdrawn from shunt 22 and catheter 12 is withdrawn from heart wall HW. Laser fiber 14 may be used at that time (or previously) to attach an upstream end of shunt 22 to heart wall HW at left ventricle LV. The laser fiber 14 and catheter 12 are then extracted from the patient. The deployed shunt 22 extends from left ventricle LV through heart wall or myocardium HW to anterior wall AW of coronary artery CA, with a middle or intermediate portion (not separately designated) of shunt 22 being disposed in intrapericardial space IS.

Fig. 2 depicts a transmyocardial coronary artery bypass similar to that shown in Fig. 1E, except that a different shunt 28 is used. Shunt 28 is provided at opposite ends with flanges 30 and 32 in the form of annular disks. These flanges 30 and 32 facilitate the attachment of shunt 28 to the heart wall HW at left ventricle LV and to anterior wall AW of coronary artery, respectively. The attachment of flanges 30 and 32 to heart wall HW and coronary artery CA may be effectuated by laser instrument 26 and/or by other techniques including gluing and suturing. Shunt 28 is installed in the manner described above with reference to Figs. 1A-1E.

The structure or shunt 28, as well as different uses thereof, is described and illustrated in U.S. Patent No. 5,470,820, the disclosure of which is hereby incorporated by reference.

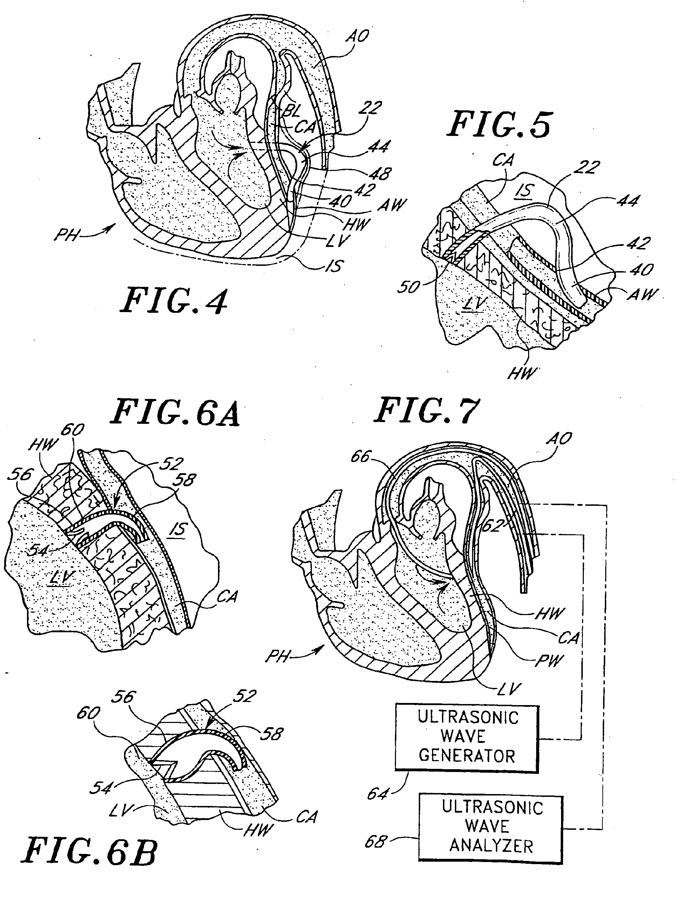

Fig. 3 shows a modification of the transmyocardial coronary artery bypass of Fig. 1 E. The downstream end of shunt 22 is attached to anterior wall AW of coronary artery CA via sutures 34. A stent 36 with a one-way valve 38 is placed inside an upstream portion of shunt 22 located within heart wall or myocardium HW. Stent 36 functions to clamp the upstream end of shunt 22 to heart wall HW. One-way valve 38 permits blood to flow from ventricle LV to coronary artery CA during systole and prevents backflow to ventricle LV during diastole. Of course, where shunt 22 is installed without stent 36, shunt 22 may be provided with an integral one-way valve (not illustrated). Stent 36 is generally introduced into heart PH in a collapsed configuration through a catheter. Stent 36 may be predisposed inside the upstream end portion of shunt 22 and inserted therewith into heart PH. Alternatively, stent 36 may be inserted into shunt 22 after the shunt has been passed through passageway 18 and before or after the attachment of the downstream end of shunt 22 to anterior wall AW of coronary artery CA. Stent 36, and other stents and shunts disclosed herein, may be provided with outwardly projecting barbs (not illustrated) for anchoring the stent or shunt to the myocardium.

In another variation (not illustrated) of the transmyocardial coronary artery bypass of Fig. 1E, shunt 22 has an upstream portion which is a stent. The stent is substantially coextensive with or smaller than passageway 18 and is accordingly lodged completely within passageway 18 upon installation of the shunt. The remainder of the shunt is made of a continuous, essentially impermeable biocompatible film material, as in the embodiments discussed above.

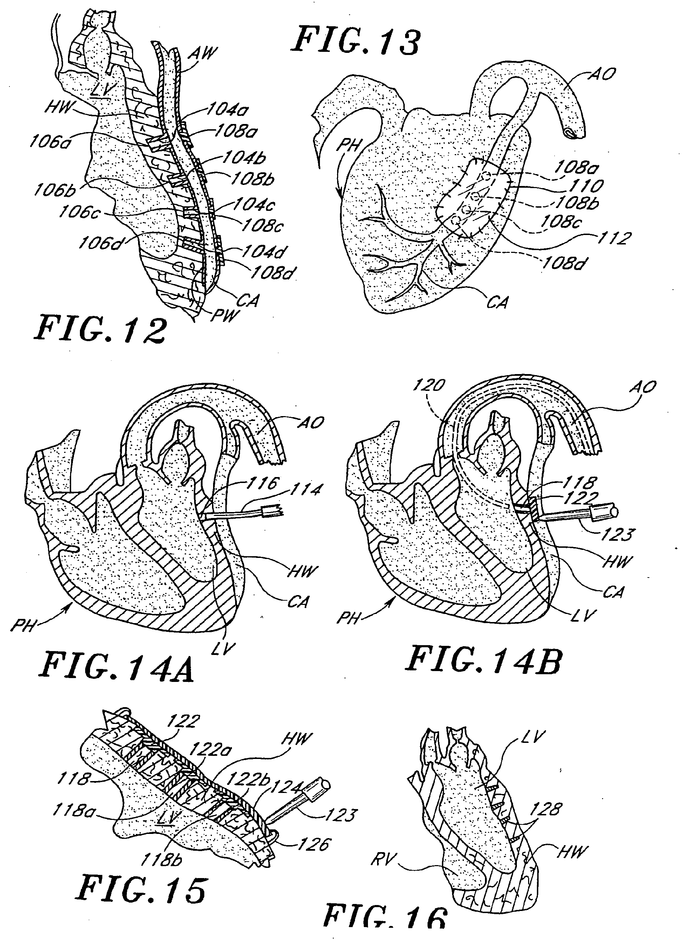

As illustrated in Fig. 4, another modification of the transmyocardial coronary artery bypass of Fig. 1E includes the insertion of a downstream end portion 40 of shunt 22 through an aperture 42 formed in anterior wall AW of coronary artery CA downstream of blockage BL. Clearly, in this bypass procedure, aperture 42 is formed in coronary artery CA prior to the joining of the downstream end portion 40 of shunt 22 and coronary artery CA. Aperture 42 is formed by an incising instrument (not shown) such as a laser or a scalpel blade which is inserted into intrapericardial space IS either through a pericardioscopic cannula or port (not shown) or through an open incision. Shunt 22 may be attached, by laser welding, glue or sutures, to coronary artery CA at aperture 42. As discussed hereinabove with respect to the embodiment of Fig. 1E, an intermediate or middle portion 44 of shunt 22 is disposed inside intrapericardial space IS upon completed deployment of shunt 22. A brace 48, for example, in the form of a patch (compare Fig. 13), may be disposed over middle portion 44 of shunt 22 and attached to heart PH, to support the shunt 22 against possible dislodgment owing to the hydraulic forces of blood flow and the mechanical forces of myocardium contraction. Brace or patch 48, and similar braces or patches disclosed herein, is made of a strong biocompatible material such as KEVLAR™, polytetrafluaroethylene, silicone, etc.

Fig. 5 illustrates the shunt-implemented transmyocardial coronary artery bypass of Fig. 4, with a one-way valve 50 being provided at the upstream end of shunt 22 for permitting blood flow from ventricle LV into coronary artery CA during systole and for preventing blood flow from coronary artery CA toward ventricle LV during diastole.

As depicted in Figs. 6A and 6B, a transmyocardial coronary artery bypass is implemented by a shunt member 52 provided at an upstream end with a one-way valve 54. Shunt member 52 extends directly from left ventricle LV through heart wall HW into coronary artery CA and includes an upstream portion 56 disposed within heart wall or myocardium HW and a downstream portion 58 disposed in coronary artery CA. Shunt member 52 may have a tapered form which narrows down in a downstream direction so that downstream portion 58 is of smaller cross-section than upstream portion 56. Upstream portion 56 may take the form of a stent which is expanded from a collapsed insertion configuration to an expanded use configuration to lock or clamp shunt member 52 to a passageway 60 formed in heart wall or myocardium HW prior to the insertion of shunt member 52. Downstream portion 58 is made of a continuous, essentially impermeable biocompatible film material. In addition, upstream portion 56 may be flexible to an extent so as to expand, if necessary, during diastole (Fig. B) to accommodate some backflow.

Shunt 52 is curved and bears the force of the blood ejected from left ventricle LV through passageway or channel 60 during systole.

As illustrated in Fig. 7, the deployment or installation of shunt 52 in an intravascular procedure requires instrumentation for enabling the precise locating of the coronary artery CA with respect to possible insertion points in left ventricle LV. To that end, a first catheter 62 is utilized which is provided at a distal end with an electroacoustic transducer (not illustrated) for converting an electrical signal of ultrasonic frequency to a mechanical pressure wave which is transmitted through a posterior wall PW of coronary artery CA and heart wall or myocardium HW. Catheter 62 and particularly the nonillustrated electroacoustic transducer is operatively connected to an ultrasonic wave generator 64. Another catheter 66 is also inserted through aorta A0 (and over a conventional guidewire, not illustrated). This second catheter 66 is introduced into left ventricle LV and is provided at a free end with an acoustoelectric transducer (not shown) for detecting pressure waves in an ultrasonic frequency range. Catheter 66 and its acoustoelectric transducer are operatively connected to an ultrasonic wave analyzer 68 which calculates the location of the distal tip of catheter 62 relative to the distal tip of catheter 66 and thus provides feedback to a surgeon or an insertion device for determining an insertion point and insertion angle for surgical incising instrument such as optical fiber 14 (Fig. 1A).

Several shunt members 22 or 52 may be necessary in cases of multiple coronary artery blockages. These multiple shunt members each tap into the coronary artery at a point downstream of a respective blockage.

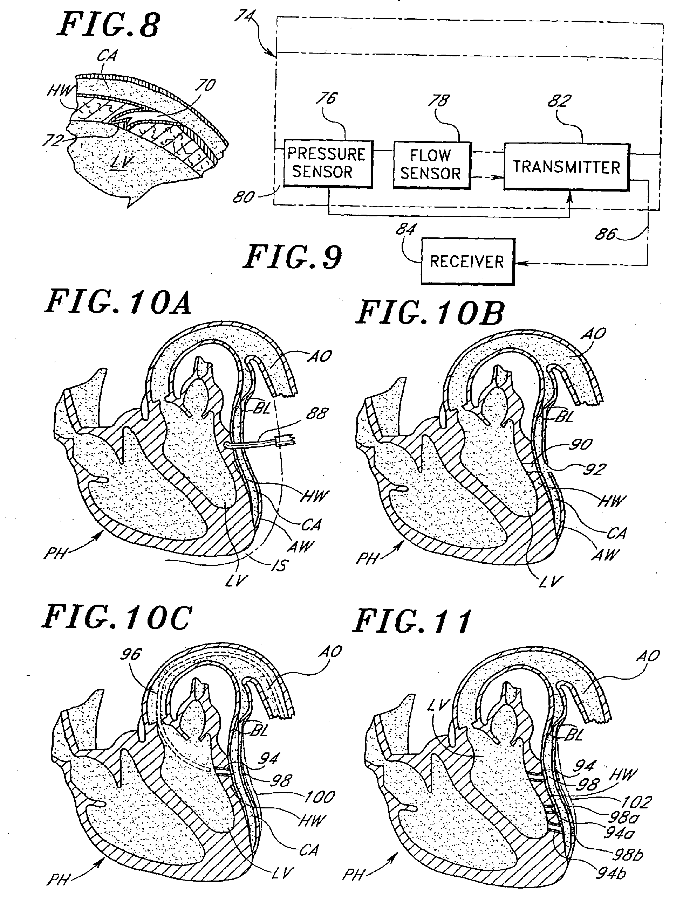

As depicted in Fig. 8, a transmyocardial stent 70 for maintaining a circulation path between left ventricle LV of patient's heart PH and coronary artery CA is curved in the longitudinal or flow direction to provide an arcuate flow path. This curvature serves to deflect the hydraulic forces from a direction substantially perpendicular to coronary artery CA to a direction substantially parallel to coronary artery CA. This deflection serves to prevent coronary artery dilatation and to protect anterior wall AW of artery CA from the substantial hydraulic forces generated during systole. Stent 70 is provided with a one-way valve 72 and may be deployed as discussed above with reference to Fig. 7. Of course, curved stent 70 may be used as upstream portion 56 of shunt member 52 or in place of stent 36 (Fig. 3) or as an upstream portion of shunt 22.

A shunt or stent 74 may be provided with a pressure sensor 76 and/or a flow sensor 78, as illustrated in Fig. 9. Sensors 76 and 78 are attached to or incorporated into a wall 80 of shunt or stent 74 and have outputs operatively connected to a transmitter 82 which is also attached to or incorporated into shunt or stent wall 80. Output signals from sensors 76 and 78 which encode date pertaining to pressures and flow rates are relayed to a receiver 84 via transmitter 82. Transmitter 82 may be wireless or connected by a wire 86 to receiver 84. The pressure and flow rate date collected via sensors 76 and 78 are useful to monitor the effectiveness of the implanted stents or shunts for any particular patient and to thereby determine whether additional stents or shunts may be necessary for that patient. Receiver 84 may be physically located on a chest of the patient or otherwise nearby.

As illustrated in Figs. 10A-10C, a transmyocardial coronary artery bypass may be performed from outside the patient's vascular system. An incising instrument 88 such as a laser or a drill is inserted pericardioscopically or through an open incision into the intrapericardial space IS and is operated to bore a passageway 90 in the heart wall or myocardium HW via the coronary artery CA, as shown in Fig. 10A. Passageway 90 (Fig. 10B) extends through heart wall HW and posterior wall PW of coronary artery CA and is aligned with an aperture 92 formed in anterior wall AW of coronary artery CA by the incising instrument 88.

Upon the formation of passageway 90, a stent 94 (Fig. 10C) is inserted in a collapsed configuration into the passageway and then expanded. Stent 94 may be inserted from outside the patient's vascular system, either through an open incision in the patient's chest or through a pericardioscopic cannula or port. Alternatively, stent 94 may be placed via a catheter 96 inserted through the vascular system including the aorta A0 and the left ventricle LV. As in all cases of stent implantation described herein, stent 94 serves to maintain passageway 90 in an open state, i.e., prevents the closure of passageway 90 by muscular contraction forces during systole and, in the longer term, by natural healing processes of the myocardium.

After the formation of passageway 90 and after the installation of stent 94 via an extravascular operation, aperture 92 is closed, via sutures (not shown) and/or via a plug or patch 98 (Fig. 10C) which is stitched or laser bonded to anterior wall AW of coronary artery CA. If stent 94 is placed via an intravascular operation, aperture 92 is preferably closed prior to the disposition of the stent inside passageway 90. A brace 100 in the form of a patch is optionally placed over plug 98 and fastened to heart PH via sutures, glue or laser welding to support the plug against possible dislodgement under blood pressure forces.

Fig. 11 illustrates a triple transmyocardial coronary artery bypass wherein a plurality of stents 94, 94a and 94b are placed in respective passageways (not separately designated) extending through heart wall or myocardium HW and posterior wall PW of coronary artery CA. The passageways are formed and the stents 94, 94a, and 94b inserted as described hereinabove with reference to Figs. 10A-10C. Plugs 98, 98a and 98b are positioned in respective apertures (not separately designated) which are formed, as discussed above, in alignment with the passageways of stents 94, 94a and 94b. A brace 102 in the form of a patch is optionally placed over plugs 98, 98a and 98b and fastened to heart PH via sutures, glue or laser welding.

Fig. 12 depicts a modification of the transmyocardial coronary artery bypass of Fig. 11, wherein passageways 104a, 104b, 104c and 104d are formed by the extravascular technique discussed above with reference to Figs. 10A-10C but which extend through posterior coronary artery wall PW and only part of the heart wall or myocardium HW from coronary artery CA. Stents 106a, 106b, 106c and 106d are inserted into respective passageways 104a, 104b, 104c and 104d via an extravascular operation. Thereafter, plugs 108a, 108b, 108c and 108d are inserted into or over respective apertures in anterior coronary artery wall AW aligned with passageways 104a, 104b, 104c and 104d and stents 106a, 106b, 106c and 106d. As illustrated in Fig. 13, a patch 110 may be placed over coronary artery CA and particularly over plugs 108a, 108b, 108c and attached via sutures 112 to heart PH to brace the plugs against dislodgement under systolic and diastolic blood pressures.

Figs. 14A and 14B depict steps in a transmyocardial revascularization procedure. An incising instrument 114 such as a laser fiber or a drill is inserted in an extravascular procedure through an open chest incision or a pericardioscopic cannula or port and used to form a channel or passageway 116 in heart wall or myocardium. HW through the epicardium (not shown). A stent 118 is inserted into channel 116 in a collapsed configuration via an intravascularly deployed catheter 120 or in an extravascular operation. A plug 122 is inserted into an outer end of channel 116 to close off that outer end. Plug 122 may be attached to the epicardium of heart PH via a laser instrument 123 or via sutures (not shown). Several channels 116, 116a and 116b may be formed and provided with respective stents 118, 118a and 118b and respective plugs 122, 122a and 122b, as illustrated in Fig. 15. A patch 124 may be placed over plug 122 or plugs 122, 122a and 122b and attached via sutures 126 to heart wall HW.

The various stents disclosed herein may be provided with a layer of polymeric material carrying a biochemical composition, e.g., angiogenesis factor or the nucleic acid instructions therefor, for generating, stimulating, and enhancing blood vessel formation. As illustrated in Fig. 16, plugs 128 may be inserted into a patient's heart wall or myocardium HW via an intravascularly deployed catheter (not shown), the plugs carrying angiogenesis factor or the nucleic acid instructions therefor for generating, stimulating, and enhancing vascular generation and growth.

It is to be appreciated that the drawings herein are schematic. The stents and shunt portions in the forms of stents described herein may have a conventional wire infrastructure not shown in the drawings. Alternatively, the stents may be made of an elastic material having an internal spring constant permitting the stent to be temporarily collapsed and able to return to an opened configuration.

Intravascular or extravascular incising instruments disclosed herein for use in forming passageways or channels in the myocardium may be contact lasers or rotating or reciprocating drills. Other instruments, in existence at the present time or introduced in the future, which are suitable for forming channels or tunnels may be used alternatively or additionally. Such instruments may take the form of ultrasonic cavitation devices, chemical devices for dissolving tissues, or heat treatment (electrocautery) devices.

Although the invention has been described in terms of particular embodiments and applications, one of ordinary skill in the art, in light of this teaching, can generate additional embodiments and modifications without departing from the spirit of or exceeding the scope of the claimed invention. For example, suturing, gluing and laser welding are discussed herein for attaching plugs and reinforcement patches or braces to the cardiac tissues. Equivalent alternatives to these techniques include stapling and tacking. Also, closure of apertures in the epicardium or coronary artery may be closed without plugs or patches, for example, by the direct application of sutures or staples or by coagulation (electrical, thermal or laser).

It is to be understood that stents are preferred to maintain open blood flow passageways in or through the myocardium. However, in some cases, stents may be omitted, for example, in the embodiments of Figs. 10C, 11, 12, 14A and 14B and 15, depending on the needs of the patient.

Generally, stent 36 (Fig. 3), upstream portion 56 (Figs. 6A, 6B) when in the form of a stent, stent 70 (Fig. 8), stents 98, 98a, 98b (Fig. 11), stents 106a- 106d (Fig. 12)," and stents 122, 122a, 122b (Fig 15) have lengths which are predetermined by measuring the thickness of the myocardium. Procedures for such measurements are described in U.S. Patents Nos. 5,429,144 and 5,662,124, the disclosures of which is hereby incorporated by reference.

Accordingly, it is to be understood that the drawings and descriptions herein are proffered by way of example to facilitate comprehension of the invention and should not be construed to limit the scope thereof.

A preferred coronary artery bypass method comprises: providing a shunt member; disposing one end portion of said shunt member in a myocardium of a patient's heart so that said one end portion communicates with a left ventricle of the patient's heart; and placing an opposite end portion of said shunt member in communication with a coronary artery of the patient downstream of a blockage in the coronary artery and so that an intermediate or middle portion of said shunt member is disposed in an intrapericardial space of the patient, outside of the myocardium and outside of the coronary artery.

In a preferred embodiment, the method further comprises attaching said opposite end portion of said shunt member to a substantially anterior wall of the coronary artery.

Preferably, the method further comprises forming an aperture in the anterior wall after attaching of said opposite end portion of said shunt member to the anterior wall, hereby opening communication between said shunt member and the coronary artery.

Preferably, the method further comprises delivering said shunt member intravascularly into the left ventricle of the patient's heart and passing said opposite end portion through the myocardium, the forming of the aperture including inserting a free end portion of an incising instrument intravascularly and through said shunt member after disposition of said one end portion of said shunt member in the myocardium and after attaching of said opposite end portion of said shunt member to the coronary artery, the forming of said aperture additionally including operating said incising instrument after inserting thereof to perforate the anterior wall of he coronary artery.

In another preferred embodiment, said incising instrument includes an optical fiber, the operating of said incising instrument including transmitting monochromatic or laser radiation through said optical fiber to the anterior wall of the coronary artery.

In a further preferred embodiment, the coronary artery bypass method further comprises forming a passageway through the myocardium prior to the disposing of said one end portion in the myocardium, said one end portion of said shunt member being disposed in said passageway.

Preferably, the forming of said passageway includes inserting a surgical instrument intravascularly into the left ventricle of the patient and operating said instrument from outside the patient to bore through the myocardium.

Preferably, said surgical instrument includes an optical fiber, the operating of said incising instrument including transmitting monochromatic or laser radiation through said optical fiber to the anterior wall of the coronary artery.

In another preferred embodiment, the placing of said opposite end portion of said shunt member in communication with the coronary artery of the patient is performed subsequently to the disposing of said shunt member in said passageway.

Preferably, the method further comprises delivering said shunt member intravascularly into the left ventricle of the patient's heart and passing said opposite end portion of said shunt member through said passageway.

In a further preferred embodiment, the method further comprises operating pericardioscopically inserted surgical instruments to manipulate said opposite end of said shunt member and to place said opposite end of said shunt member into communication with the coronary artery of the patient after passing of said opposite end portion of said shunt member through said passageway.

Preferably, the method further comprises inserting said opposite end portion of said shunt member into the coronary artery.

Preferably, the method further comprises delivering said shunt member intravascularly into the left ventricle of the patient's heart and passing said opposite end portion through the myocardium, the inserting of said opposite end portion of said shunt member into the coronary artery being performed subsequently to the passing of said opposite end portion of said shunt member through the myocardium.

Preferably, the method further comprises operating pericardioscopicaby inserted surgical instruments to manipulate said opposite end of said shunt member and to place said opposite end of said shunt member into communication with the coronary artery of the patient after passing of said opposite end portion of said shunt member through the myocardium.

In a further preferred embodiment, the method further comprises delivering said shunt member intravascularly into the left ventricle of the patient's heart and passing said opposite end portion through the myocardium.

Preferably, the method further comprises bending said shunt member after passing of said opposite end portion through the myocardium.

Preferably, the method further comprises operating pericardioscopically inserted surgical instruments to manipulate said opposite end of said shunt member and to place said opposite end of said shunt member into communication with the coronary artery of the patient.

In a preferred embodiment, a shunt for implantation into a patient to effectuate a coronary artery bypass comprises a generally tubular member having a bend between opposite ends of said tubular member so that blood traversing said shunt upon implantation thereof at least partially in a myocardium of the patient travels along an arcuate path, said tubular member having a length greater than a width of the myocardium.

Preferably, said tubular member of the shunt is flexible at least along a middle or intermediate portion thereof.

In another preferred embodiment, said tubular member of the shunt is provided with a one-way valve.

In a further preferred embodiment, said tubular member of the shunt is wider at an upstream one of said opposite ends than at a downstream one of said opposite ends.

In a preferred embodiment, a coronary artery bypass method comprises: providing a generally tubular shunt member; disposing an upstream end portion of said shunt member in a myocardium of a patient's heart so that said one end portion communicates with a left ventricle of the patient's heart, and inserting a downstream end portion of said shunt member into a coronary artery of the patient downstream of a blockage in the coronary artery so that said downstream end portion is disposed inside said coronary artery.

Preferably, the method further comprises delivering said shunt member intravascularly into the left ventricle of the patient's heart and passing said downstream end portion through the myocardium.

Preferably, said downstream end portion is inserted directly into the coronary artery through a posterior wall thereof in contact with the myocardium.

In an alternative preferred embodiment, said downstream end portion is inserted into the coronary artery through a substantially anterior wall thereof, so that an intermediate or middle portion of said shunt member is disposed in an intrapericardial space of the patient, outside of the myocardium, and outside of the coronary artery.

Preferably, said upstream end portion of said shunt member is disposed in the myocardium and said downstream member is inserted into the coronary artery is that an intermediate or substantially middle portion of said shunt member is bent or curved between said upstream end portion and said downstream end portion so that blood traversing said shunt member upon implantation thereof in the patient travels along an arcuate path.

In a further preferred embodiment, a method for performing a myocardial revascularization, comprises: forming a passageway at least partially through a myocardium of a patient from an outer surface of the patient's heart; and performing a surgical operation at an outer end of said passageway to permanently close said passageway at said outer end.

Preferably, the forming of said passageway includes forming an aperture in an anterior wall of a coronary artery of the patient, the passageway being formed in substantial alignment with said aperture, the performing of said surgical operation including closing said aperture in the anterior wall of the coronary artery.

Preferably, the method further comprises inserting a stent into said passageway.

Preferably, the inserting of said stent is performed prior to the performing of said surgical operation to close said passageway at said outer end.

Preferably, the closing of said aperture includes suturing the anterior wall of the coronary artery to close said aperture.

Preferably, said passageway is formed to communicate at an inner end with a left ventricle of the patient.

In an alternative preferred embodiment, said passageway is formed to terminate in the myocardium of the patient and to communicate at an outer end with the coronary artery.

Preferably, the forming of said aperture and the forming of said passageway including operating an instrument taken from the group consisting of a surgical drill and a surgical laser.

Preferably, said passageway is one of a plurality of passageways extending from the coronary artery into the myocardium of the patient, further comprising forming a plurality of openings in the anterior wall of the coronary artery and forming said passageways in alignment with respective ones of said openings, said aperture being one of said openings, also comprising closing said openings in the anterior wall of the coronary artery.

In a further preferred embodiment, the method for performing a myocardial revascularization, further comprises surgical attaching a brace element to the patient's heart over the coronary artery and said passageway, thereby supporting said coronary artery against hydraulic pressures generated by the heart.

Preferably, the method further comprises inserting a stent into said passageway.

Preferably, the inserting of said stent is performed prior to the performing of said surgical operation to close said passageway at said outer end.

Preferably, the forming of said passageway includes operating an instrument taken from the group consisting of a surgical drill and a surgical laser.

Preferably, said instrument is a pericardioscopic instrument, further comprising inserting a distal end of said instrument into an intrapericardial space of a patient through a cannula.

Preferably, the surgical operation is performed on an epicardium of the patient.

Preferably, the surgical operation includes inserting a plug into said passageway at said outer end thereof.

In another preferred embodiment, a stent has a collapsed configuration and an expanded configuration, said expanded configuration having an arcuate form, to provide a curved flow path for blood upon implantation of said stent into a myocardium of a patient.

In a further preferred embodiment, a stent has a collapsed configuration and an expanded configuration, said stent being provided with a sensor and means for transmitting signals from said sensor to a receiver external to the stent.

Preferably, said sensor is taken from the group consisting of a pressure sensor and a flow sensor.

## Claims

1. A stent having a collapsed configuration and an expanded configuration, said expanded configuration having an arcuate form to provide a curved flow path for blood upon implantation of said stent into a myocardium of a patient.

2. A stent having a collapsed configuration and an expanded configuration, said stent being provided with a sensor and means for transmitting signals from said sensor to a receiver external to the stent.

3. A stent for implantation into tissue, the stent having a first collapsed configuration and a second expanded configuration and configured to be inserted into a heart wall between a heart chamber and a coronary vessel; and an angiogenic substance associated with the implant.

4. The stent as defined in claim 3, wherein the stent defines a hollow interior.

5. The stent as defined in claim 3, wherein the stent is provided with a layer carrying the angiogenic substance.

6. The stent as defined in claim 3, further comprising an angiogenic substance loaded onto the stent prior to implantation of the stent.

7. The stent as defined in claim 3, wherein the stent is a cylinder.

8. The stent as defined in claim 3, wherein the stent is resiliently expandable.

9. The stent as defined in claim 3, wherein the stent is flexible after assuming the second expanded configuration.

10. The stent as defined in claim 3, wherein the angiogenic substance is for generating, stimulating, and/or enhancing blood vessel formation.

11. The stent as defined in claim 3, wherein the stent is configured to be delivered to the heart wall intravascularly.

12. The stent as defined in claim 3, wherein the stent includes outward projections for anchoring the implant.

13. The stent as defined in claim 3, wherein an end of the stent includes a flange.

14. The stent as defined in claim 3, wherein the stent includes a portion configured to be positioned within the coronary vessel.

15. The stent as defined in claim 14, wherein the stent includes a bend.
